# EUROPEAN PATENT APPLICATION

(11) **EP 4 710 951 A1**
(43) Date of publication of application: **18.03.2026**
(21) Application number: 24382981.9
(22) Date of filing: 13.09.2024
(51) Int. Cl.: A61L 2/08, A01K 1/00, A61L 2/22

(54) **METHOD FOR DISINFECTING LIVESTOCK FACILITIES**

(71) Applicant: Active Walls, S.L., 08100 Mollet Del Valles (Barcelona) (ES)
(72) Inventor: SERRA MOTAS, Jesús, 08100 MOLLET DEL VALLES (Barcelona) (ES)
(74) Representative: Herrero & Asociados, S.L.

(57) **Abstract**

The present invention relates to a method for disinfecting livestock facilities, particularly those used for housing and breeding. The disinfection method has a sustained effect over time.

## Description

### FIELD OF THE INVENTION

The present invention relates to a method for disinfecting livestock facilities, particularly those used for housing and breeding. The disinfection method has a sustained effect over time.

### BACKGROUND OF THE INVENTION

Current methods for disinfecting livestock facilities involve a variety of chemical and physical approaches aimed at controlling and eliminating harmful pathogens. Traditional chemical disinfectants, such as bleach and quaternary ammonium compounds, are widely used due to their effectiveness in killing a broad spectrum of microorganisms. However, these chemicals often leave harmful residues, require significant labour, and can pose risks to both animal and human health. Physical methods, including highpressure washing and thermal treatments, are also employed to remove contaminants, but these techniques can be labour-intensive and may not reach all surfaces effectively.

The document EP0480841 describes a disinfectant composition, characterised in that it comprises, as active ingredient of the formula R-X-CH₂-CHOH-CH₂NH₂ in which X is - O-, -S-, -NH- or -CH₂-; R is a straight-chain aliphatic C₅ to C₁₈-radical. This disinfectant composition is aimed at the food industry and veterinary medicine.

Maintaining hygiene and preventing the spread of diseases in livestock facilities is crucial for animal health and productivity. Traditional methods of disinfection often involve chemical agents that may leave harmful residues and require extensive labour.

Recently, there has been growing interest in more sustainable and environmentally friendly disinfection technologies, such as the use of ultraviolet (UV) light and hydrogen peroxide vapor. These methods offer the potential for effective pathogen control without the drawbacks associated with traditional chemical disinfectants. Despite these advancements, there remains a need for improved disinfection methods that are both effective and safe for use in livestock environments.

### SUMMARY OF THE INVENTION

The method of disinfection describes in the present invention offers numerous advantages for livestock facilities. It continuously eliminates all types of microorganisms on farms, effectively reducing the presence of bacteria, viruses, and fungi. Additionally, it breaks down ammonia and purifies the environment, leading to a significant reduction in unpleasant odors. By removing organic chemical compounds from the air, it ensures a cleaner and healthier atmosphere. This eco-friendly and non-polluting technology also lowers levels of ammonia, nitrogen oxides, carbon monoxide, hydrogen sulfide, and odorous compounds such as amines and amides. The reduction of these harmful components notably decreases exposure to disease-causing microorganisms, particularly those affecting the respiratory system, thereby promoting better overall health for both animals and workers.

The method comprises a step wherein a TiO₂ aqueous dispersion is applied to the different surfaces to be treated, theses surfaces could be of any type of material like, concrete, wood, plastic and metal. Finally, the surfaces are exposed to high energy visible light (HEV). The object of these steps is to maintain along the time the disinfection. When the surfaces are exposed to HEV, the photocatalysis process of titanium dioxide (TiO₂) starts, this photocatalysis process is an oxidation mechanism used to decompose organic pollutants. This process is activated under exposure to HVE, which has a wavelength sufficient to excite TiO₂, promoting the generation of reactive species. When the organic matter, which contains C-H and C-O bonds, comes into contact with these reactive species, the bonds are attacked and decomposed, resulting in the formation of carbon dioxide (CO₂) and water (H₂O). Also, when titanium dioxide (TiO₂) is photoexcited, it can effectively destroy ammonia (NH₃) and other nitrogen compounds and microorganisms through a series of reactions involving reactive oxygen species.

With the method of the invention, all types of microorganisms in farms are continuously eliminated, with proven efficacy against *Escherichia coli, Staphylococcus aureus, Brachyspira hyodysenteriae, Salmonella spp., Enterobacteriaceae,* aerobic mesophiles, *Adenovirus type 5*, fungi, and yeasts, among others. The method decomposes ammonia, purifies the environment, eliminates odors, and removes organic chemical compounds from the air. It reduces levels of ammonia, nitrogen oxides, carbon monoxide, hydrogen sulfide, and odorous compounds such as amides, amines, and sulfides. Thanks to the reduction of these components, exposure to disease-causing microorganisms is significantly decreased, especially those affecting the respiratory system.

Therefore, an aspect of the invention is a method of the invention for disinfecting livestock facilities comprising the steps of: (b) applying a TiO₂ aqueous dispersion to the surfaces to be disinfected wherein the aqueous dispersion comprises TiO₂ in a range from 0.1% w/wt to 15% w/wt; (c) exposing the surfaces to high energy visible light.

The term "%w/vnt" means the percentage of the compound cited respect to the total weight of the dispersion.

The term high-energy visible light (HEV light) means a portion of the visible light spectrum with wavelengths ranging from 350 to 500 nanometers. This type of light is known for its higher energy levels compared to other visible light and is often associated with blue and violet hues. One of the significant advantages of this light is its lack of toxicity to animals, ensuring their safety while maintaining a clean environment. Additionally, it is compatible with day and night cycles, allowing for continuous use without disrupting natural routines. This makes it an ideal solution for spaces that require constant sanitation without compromising the health and well-being of animals.

The TiO₂ aqueous dispersion used in this method is a non-toxic, non-hazardous active substance. It is non-flammable and does not form a film on surfaces. Additionally, the dispersion is translucent, ensuring that it does not alter the appearance of the treated areas. A key benefit of this disinfectant is its lack of toxicity to animals, ensuring their safety while maintaining a clean environment. In contrast to traditional disinfectants, this product can be applied even in the presence of animals, which makes it highly convenient and safe.

In the present invention the term livestock facilities mean structures and spaces designed for the housing, breeding, feeding, and care of domesticated animals raised for agricultural purposes, such as cattle, pigs, sheep, poultry, and other livestock. These facilities include barns, stables, pens, coops, feedlots, and other enclosures equipped with necessary amenities to ensure the health, safety, and productivity of the animals. Additionally, the system can be applied to surfaces related to the treatment of animals, such as slaughterhouses and transportation vehicles. Furthermore, it can be used on all farm surfaces related to biosecurity, including locker rooms, storage areas, access points, and even courtyards.

### DETAILED DESCRIPTION OF THE INVENTION

As it has explained above the present invention refers to a method of the invention for disinfecting livestock facilities comprising the steps of: (b) applying a TiO₂ aqueous dispersion to the surfaces to be disinfected wherein the aqueous dispersion comprises TiO₂ in a range from 0.1% w/wt to 15% w/wt; (c) exposing the surfaces to high energy visible light.

Examples of surfaces in livestock facilities include floors, including corridors and areas where there is potential for pathogen transfer, partitions, equipment that comes into contact with animals, walls, and changing rooms.

In a preferred embodiment before the step b) there is a step a) cleaning the surfaces of the livestock facilities to be disinfected, particularly with an alkaline detergent.

In a preferably embodiment the TiO₂ range is between 6% w/wt to 12% w/wt. In a particular embodiment the TiO₂ range is between 6% w/wt and 6.5% w/wt. In other particular embodiment the TiO₂ range is between 8% w/wt and 10% w/wt. Finally, it is other particular embodiment the TiO₂ range is between 10% w/wt and 12% w/wt.

Preferably the TiO₂ aqueous dispersion comprises a binder in a range from 0.1%w/wt to 5%w/wt. In a particular embodiment the binder is an alkaline silicate in particular sodium or potassium silicate.

In other preferred embodiments of the invention high energy visible light comes from LED lamps. In a more preferred embodiment, the wavelengths ranging from 350 to 390 nanometers. In other preferred embodiment the LED lamps the power density is in a range from 0.01 Watts/m² to 0.2 Watts/m², this means than the power received in the exposing surface.

The TiO₂ aqueous dispersion shows a density in a range from 1 kg/l to 1.3 kg/l; particularly 1.1 kg/l.

Preferably the application is a spray application. The spray application yield is in a range between 10 m²/l to 50 m²/l. In a preferably embodiment the step of application is carried out at a temperature range between 5°C and 35°C.

Preferably the application is in one time and in a range from 100 g/m² to 20 g/m². In a preferred embodiment the high energy visible light comes from a light emitted diode (LED).

### Examples

The present invention will be described in more detail with reference to the following examples, but not only limited to these described functions.

### Example 1

In an example of the invention, a poultry farm was disinfected. LED lamps operating at 24 V DC with a daily consumption of 26W were installed. They were positioned at a height of 2 meters above the farm floor and spaced 5 meters apart. The goal was to achieve regular illumination with minimal shadows, ensuring light reached all areas of the farm in contact with the animals, which are susceptible to containing or spreading infections. The lamps were 490 mm in length, with six diodes in a rigid aluminum structure, an intensity of 1.2 A, and an emission of 5 Watts with a 120° emission angle.

The farm floor, was sprayed with a dispersion containing 6 %w/w dispersion, at a ratio of 10 m²/ liter, covering the floor and the walls at 1,5 m high (animal reach high)

The farm had a previous and recurrent infection of salmonella that couldn't be eliminate using traditional disinfection methods. One week after the application of the invention, salmonella was not detected in lab analysis, for first time in years

### Example 2

In an example of the invention, a pig farm was disinfected. LED lamps operating at 24 V DC with a daily consumption of 26W were installed. They were positioned at a height of 2,5 meters above the farm floor and spaced 5 meters apart.

The farm had a previous infection of pig dysentery, caused by the bacteria named Brachyspira. The infection is, in normal conditions, continuously increasing, because the bacteria reproduce and survives in the manure, reinfecting healthy and ill animals.

The farm floor was sprayed with a dispersion containing 6 %w/w dispersion, at a ratio of 10 m²/ litter, covering the floor and the walls at 1,5 m high (animal reach high). One week after the application of the invention, Brachyspira was not detected in significant quantities in the treated areas, and average animal health was clearly improved

## Claims

1. A method for disinfecting livestock facilities **characterized by** comprising the steps of: (b) applying a TiO₂ aqueous dispersion to the surfaces to be disinfected, wherein the aqueous dispersion comprises TiO₂ in a range from 1% w/wt to 15% w/wt; (c) exposing the surfaces to high energy visible light.

2. The method according to the claim 1 **characterized by** the TiO₂ range is between 6% w/wt and 6.5% w/wt.

3. The method according to any one of the claims 1 to 2 **characterized by** the TiO₂ aqueous dispersion comprises a binder in a range from 0.1%w/wt to 5%w/wt.

4. The method according to claim 3 **characterized by** the binder is an alkaline silicate.

5. The method according to the claim 4 **characterized by** the alkaline silicate is selected from sodium or potassium silicate.

6. The method according to any one of the claims 1 to 5 **characterized by** TiO₂ range is between 10% w/wt and 12% w/wt.

7. The method according to any one of the claims 1 to 6 **characterized by** further comprising a step a) of: a) cleaning the surfaces of the livestock facilities to be disinfected.

8. The method according any one of the claims 1 to 7 **characterized by** in the step c) the wavelengths of the high energy visible light ranging from 350 to 390 nanometers.

9. The method according any one of the claims 1 to 8 **characterized by** the high energy visible light comes from LED lamps.

10. The method according to the claim 9 **characterized by** the power received in the exposing surfaces from the LED lamps is in a range from 0.01 Watts/m² to 0,2 Watts/m².

11. The method according any one of the claims 1 to 10 **characterized by** the application of step b) is a spray application.

12. The method according to the claim 11 **characterized by** the spray application yield is in a range between 10 m²/l to 50 m²/l.

13. The method according any one of the claims 1 to 12 **characterized by** the step of application b) is carried out at a temperature range between 5°C and 35°C.

14. The method according any one of the claims 1 to 13 **characterized by** the application b) is in one time and in a range from 100 g/m² to 20 g/m².
